## Europäisches Patentamt
## European Patent Office
## Office européen des brevets

(19)

(11) Veröffentlichungsnummer: **0 137 833**
**B1**

(12) **EUROPÄISCHE PATENTSCHRIFT**

(45) Veröffentlichungstag der Patentschrift:
**07.01.88**

(51) Int. Cl.⁴: **C 02 F 11/04, C 12 M 1/00, C 12 P 5/02**

(21) Anmeldenummer: **84901210.9**

(22) Anmeldetag: **19.03.84**

(86) Internationale Anmeldenummer:
**PCT/HU 84/00017**

(87) Internationale Veröffentlichungsnummer:
**WO 84/03694 (27.09.84 Gazette 84/23)**

(54) **EINRICHTUNG ZUR KONTINUIERLICHEN UND NASSEN FERMENTATION FÜR LANDWIRTSCHAFTLICHE NACHPRODUKTE UND ABWASSERSCHLAMMEN DURCH ANWENDUNG VON AUS GASDICHTEM KUNSTSTOFF HERGESTELLTEN BEHÄLTERN.**

(30) Priorität: **25.03.83 HU 100783**

(43) Veröffentlichungstag der Anmeldung:
**24.04.85 Patentblatt 85/17**

(45) Bekanntmachung des Hinweises auf die Patenterteilung:
**07.01.88 Patentblatt 88/1**

(84) Benannte Vertragsstaaten:
**AT BE CH DE FR GB LI LU NL SE**

(56) Entgegenhaltungen:
**DE - A - 2 407 759**
**DE - A - 3 207 048**
**DE - C - 627 114**
**DE - C - 640 367**
**FR - A - 2 474 474**
**US - A - 4 401 565**

**Biogasanlagen in Europa, Verlag TÜV Rheinland GMBH, KÖLN (1985), Seiten 27,29,41,50**

(73) Patentinhaber: **ENERGIAGAZDALKODASI INTEZET, 33-34, Bem-rakpart, H-1027 Budapest II (HU)**
Patentinhaber: **Rozmaring Fejlesztési Innováciòs Mezögazdasági Szakcsoport, H-2083 Solymár (HU)**
Patentinhaber: **GRABOPLAST Pamutszövö és Mübörgyár, Fehérvari ut 16/B, H-9002 Györ (HU)**
Patentinhaber: **Kisbéri "Virágzo" Mezögazdasági Termelöszövetkezet, H-2870 Kisbér (HU)**

(72) Erfinder: **BARTHA, István, Váci ut 41., H-1056 Budapest (HU)**
Erfinder: **DENKE, László, Sólyom ut 16/b., H-1126 Budapest (HU)**
Erfinder: **RIDEG, György, KOVACS, Kalman, H-1081 Budapest (HU)**
Erfinder: **KOVACS, Kálmán, Kassa köz 7/a., H-1142 Budapest (HU)**
Erfinder: **CSAPLINSZKY, László, Csalogány ut 54., H-2030 Erd (HU)**
Erfinder: **IGAZVÖLGYI, Csaba, Szövo ut 22., H-9000 Gyor (HU)**
Erfinder: **WAPPEL, Kálmán, Otthon ut 7., H-9000 Gyor (HU)**
Erfinder: **FÜZY, Péter, Rákóczi ut 91., H-9343 Beled (HU)**
Erfinder: **LEHOTZKY, Györgyné, Mádi ut 91., H-1104 Budapest (HU)**
Erfinder: **TÖRÖK, Pál, Balogh Adám ut 9., H-1026 Budapest (HU)**

(74) Vertreter: **Patentanwälte Viering & Jentschura, Steinsdorfstrasse 6, D-8000 München 22 (DE)**

## Beschreibung

Die Erfindung betrifft eine Einrichtung zur kontinuierlichen und nassen Fermentation landwirtschaftlicher Nachprodukte und von Abwasserschlämmen zwecks Herstellung von Biogas bzw. nährstoffhaltigem flüssigen organischen Dünger, mit wenigstens einem Gärbehälter, dessen Gasaustrittsleitungen mit wenigstens einem Trockengasbehälter in Verbindung stehen, wobei das ausfermentierte Material aus dem Gärbehälter in einen geschlossenen Biodüngerbehälter geleitet wird.

Es ist wohlbekannt, dass aus Tierdünger und landwirtschaftlichen, eventuell Nebenprodukten der Lebensmittelindustrie und den auch bei der Behandlung von Abwässern entstammenden Schlämmen Biogas durch verschiedene Verfahren bzw. durch Anwendung von aus Stahl oder Stahlbeton hergestellten Konstruktionen erzeugt werden. Die Biogase bzw. in vielen Fällen auch die Endprodukte der Gärung und die aus Ausfaulverfahren zurückbleibenden Schlämme werden häufig zur Herstellung von flüssigen organischen Düngern verwendet.

In der bisherigen Praxis – wie schon oben erwähnt – werden zur Herstellung eines flüssigen organischen Düngers – ausgehend von den in der Abwasserreinigung verwendeten Anlagen – aus Stahlbeton oder Stahl hergestellte Gärbehälter bzw. Ausfaulbehälter, Gasbehälter und Behälter für flüssigen Biodünger verwendet.

Nach der Fachliteratur sind Einzelheiten über die erwähnten Stahl- bzw. Stahlbetonkonstruktionselemente unter anderen in den nachstehenden Publikationen erwähnt:

1) Kleinhaus W.: Wirtschaftlichkeit eines aus tierischem Mist erzeugten Biogases (Berichte über Landwirtschaft, Hamburg/Berlin, 1980, Band 4, Seiten 560–595).

2) Göbel W.: Vergleich der verschiedenen Biogasanlagen (schweizerische landwirtschaftliche Monatshefte, Bern, 1980, Band 8/9, Seiten 349–362)

3) Englert G.: Die optimale Wärmeisolation der Biogasanlagen (Grundlagen der Landtechnik, Düsseldorf, 1981, Band 3, Seiten 77–80)

4) Barades W., Dohne E., Brenndürfer M.: Biogas in Theorie und Praxis (Kuratorium für Technik und Bauwesen in der Landwirtschaft, Darmstadt, 1978, Seite 134)

5) D.K. Subromian and Co.: Optimalisierung der Biogasanlagen (Wissenschaftliches Institut Indiens, Bangalore, 1979, Band VI. 20, Seite 20).

Das gemeinsame Merkmal der in den oben erwähnten Publikationen dargelegten Ausführungsformen besteht darin, dass die Anlagen, die zur Herstellung des Biogases und organischen Düngers mit erhöhter Wirtschaftlichkeit dienen, alle aus Stahl und aus Stahlbeton hergestellt sind, so dass diese jedenfalls individuell konstruiert bzw. hergestellt werden müssen. Demzufolge ist eine Serienproduktion zu kompliziert und wäre auch zu kostspielig. Ein weiterer Nachteil dieser Konstruktionen liegt darin, dass ihr Konstruktionsmaterial aus Stahl und Eisenbeton ist, die im Laufe des Betriebs bzw. des Verfahrens leicht und schnell korrodieren, wofür man für einen besonderen Korrosionsschutz sorgen muss, der immer wieder zu erneuern ist.

Es ist weiter nachteilig, dass im Verlaufe des technologischen Verfahrens der unentbehrliche Materialtransport (Einfüllen, Entleeren, usw.) wegen der starren Konstruktion sehr schwierig und arbeitsaufwendig ist, so dass man deswegen spezielle Einfüll- bzw. Entleerungseinrichtungen verwenden muss.

Es ist auch ein Faulturmbehälter bekannt (DE-A-24 07 759), der einschliesslich seines auf einem Betonfundament aufliegenden Bodens aus einem biegeschlaffen Material, wie einem Kunststoffmaterial ausgebildet ist. Dieser Faulbehälter ist jedoch von einem turmartigen Stahlgerüst umgeben. Ferner ist es bekannt (DE-A-32 07 048), die Wanne und den Deckel eines Digestors für die Fermentation von Zellulosematerialien aus Beton bzw. Metall oder aus einem Kunststoffmaterial herzustellen.

Im Sinne der Erfindung verwenden wir für die Gärbehälter und Ausfauler, weiter für die Gasbehälter und Biodüngerbehälter solche aus gasdichten Kunststoff-Folienelementen hergestellten Behälter, deren Elemente aus Kunststoffmaterial handelsüblich erhältlich sind. Diese gasdichten Kunststoffbehälterbesitzen den grossen Vorteil, dass sie an dem Anwendungsort in Form einer erfindungsgemässen Konstruktion schnell und mit geringem Kostenaufwand aufstellbar sind. Falls diese aus diesem gasdichten Kunststoff hergestellten Elemente eventuell beschädigt werden, können sie am Verwendungsort sehr einfach und mit einfachen Mitteln ausgebessert werden und die eventuell schon verbrauchten Elemente aus diesem gasdichten Stoff können binnen ein bis zwei Tagen gegen vorfabrizierte Elemente ausgetauscht werden.

Durch diese Massnahmen können wir die bisherigen Investitionskosten um 40–50% herabsetzen und können günstigere Betriebszustände dadurch erreichen, dass diese Gärbehälter und Ausfaulerkonstruktionen aus gasdichtem Kunststoffmaterial keinen Sonderkorrosionsschutz beanspruchen, und weiter, dass eine ständige Aufrechterhaltung und Erneuerung des Korrosionsschutzes völlig entfallen kann. Die Ausbildung des Gasbehälters ist an sich wasserverschlusslos, so dass gegen Frostgefahr keine Sondermassnahmen getroffen werden müssen, und man für die Heizung des herkömmlichen Wasserverschlusses, die Anwendung eines Ölverschlusses usw. keine Sorgen tragen muss.

Die Ausbildung der Einrichtung gemäss der Erfindung beruht auf der Erkenntnis, dass heutzutage ein Kunststoffmaterial mit hoher Temperaturfestigkeit (zwischen −30 °C und +80 °C) und einem sehr hohen Belastbarkeitsvermögen handelsüblich erhältlich ist, d.h. die bzw. aus diesem Kunststoffmaterial hergestellten Elemente, miteinander verschweisst, bei der nassen Biogas-Biodünger-Herstellung auch den sehr hohen Belastungsansprüchen gut entsprechen, leicht serienmässig herstellbar sind und durch Verschweissen sehr haltbar miteinander verbunden werden können. Im Falle einer Beschädigung können sie leicht ausgebessert werden. Es ist auch zu betonen, dass infolge bei den Biogas-Biodünger-Herstellungsverfahren im Laufe ihrer Gär- und Ausfaulerverfahren weder ihr Belastbarkeitsvermögen noch ihre anderen technischen Parameter nachteilig schlechter werden. Die unsererseits vorgeschlagenen gasdichten Kunststoffmaterialien sind wegen ihren physikalischen Eigenschaften äusserst fähig dazu, dass aus diesen Kunststoffmaterialien Dünger- bzw. Gärbehälter, Trockengasbehälter und Flüssigbiodüngerbehälter individuell oder in vorgefertigter Serienproduktion usw. in verschiedenen typisierten Abmessungen hergestellt werden können, so dass sie in einem erprobten und garantierten Zustand guter Qualität am Ort der Anwendung aufstellbar bzw. montierbar sind. Diese Behälter können für die konventionellen Betriebszwecke an weitere Konstruktionselemente angeschlossen werden, d.h. diese Kunststoffbehälter bilden mit diesen weiteren Elementen zusammen eine komplette Biogasanlage.

Die Erfindung betrifft eine Einrichtung zur kontinuierlichen und nassen Fermentation landwirtschaftlicher Nachprodukte und von Abwasserschlämmen. Die Einrichtung verfügt über wenigstens einen Gärbehälter, dessen Gasaustrittsrohre mit wenigsten einem Trockengasbehälter verbunden sind, wobei der Gärbehälter für den Transport des Ausgärungsproduktes an einen geschlossenen Biodüngerbehälter angeschlossen ist.

Das Wesen der Erfindung liegt darin, dass diejenigen Konstruktionselemente des Gärbehälters, des Trockengasbehälters und des geschlossenen Flüssigbiodüngerbehälters, die mit dem zu fermentierenden Rohmaterial, mit dem Biogas bzw. dem Fertigprodukt-Biodünger in Berührung stehen, aus gasfestem Kunststoffmaterial ausgebildet sind, wobei die Behälter mit ihrer unteren Hälfte in den Erdboden eingebettet sind, der schräg angeordnete Gärbehälter mit seinem oberen und unteren Ende an oberen Zuführ- bzw. unteren Abführkanalwänden festgelegt ist und die Kunststoffwände des Trockengasbehälters und des Biogasbehälters an Ringträgern verankert sind. Das Volumen jedes Gärbehälters wird durch die Festigkeitsparameter des gasdichten Kunststoffmaterials bestimmt, so dass gegebenenfalls mehrere Gärbehälter zueinander parallelgeschaltet angeordnet sind.

Im Sinne einer vorteilhaften Ausführungsform der Einrichtung gemäss der Erfindung ist das eine Ende des Gärbehälters an der Wand eines Kanals der öffentlichen Werke durch Einhängung und weiter durch verschiedene Schlamm-, Gas- und Rauchrohre, sein anderes Ende jedoch an der Wand eines Schlamm ableitenden Kanals mittels eines Ausleitungsrohres verankert. Die Gärbehälter weisen ein Mannloch auf, durch welches hindurch Stützen aufgestellt werden, die die abmontierbaren Rohrleitungen fixieren.

In der unmittelbaren Nähe dieser Stützen sind Heizorgane, z.B. Rohrschlangen angeordnet.

Der Trockengasbehälter verfügt über je eine untere und obere Kunststoffmembran, deren Rand an dem Ringbalken fixiert ist.

Die Bodenplatte ist an einer Stahlbetongrundplatte'fixiert.

Der obere Teil dieses Trockengasbehälters ist an einer als Druckplatte dienenden Stahldruckplatte fixiert, die selbst mit einer eine funkenfreie Bewegung gewährleistenden Führungsstange und einer diese letztere umfassenden Rahmenkonstruktion verbunden ist.

Der geschlossene Flüssigbiodüngerbehälter besteht aus einer unteren Membran und einer oberen Membran, die zusammen mit einem Ringbalken in einem rechteckigen Erdaufwurf festgehalten sind. In ihrer einen Seite ist eine aus einer Doppelfolie ausgebildete Öffnung vorhanden, die zum Entfernen der ausgereiften organischen Dünger dient.

Die Einrichtung gemäss der Erfindung wird anhand eines Ausführungsbeispieles, dargestellt in der Zeichnung, näher erläutert, wobei die

Fig. 1 die allgemeine schematische Anordnung der Einrichtung gemäss der Erfindung in Draufsicht darstellt;

Fig. 2 den aus gasdichtem Kunststoffmaterial hergestellten Gärbehälter in der Erde eingebettet, an einem Kanal der öffentlichen Werke angeschlossen, und zwar im Längsschnitt, schematisch darstellt;

Fig. 3 den Schnitt entlang der Linie X–X in Fig. 2 zeigt;

Fig. 4 den Trockengasbehälter, hergestellt im Sinne der Erfindung aus gasdichtem Kunststoffmaterial, und zwar versehen mit einem Luftraum und angeordnet in einer isolierten Kunststoffumhüllung mit Stahlskelett, im Längsschnitt schematisch darstellt;

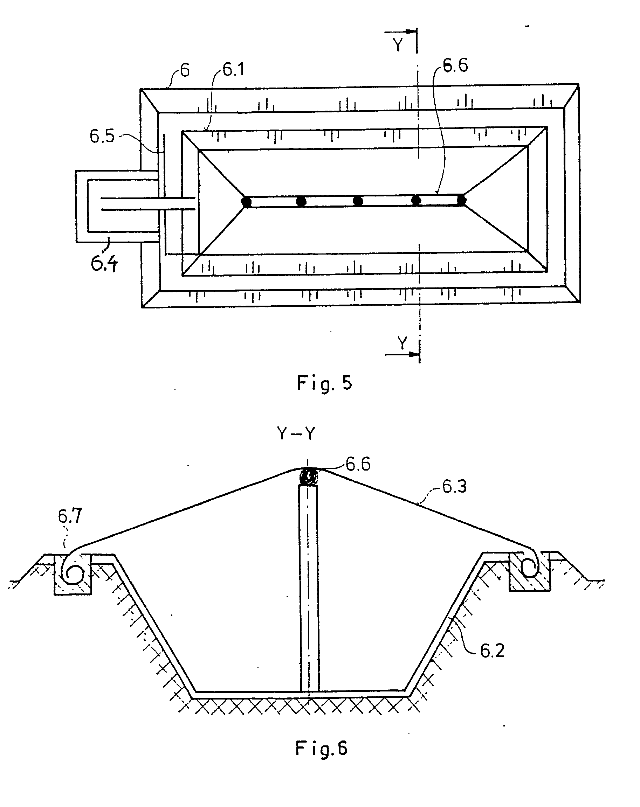

Fig. 5 den Flüssigbiodüngerbehälter, hergestellt aus gasdichtem Kunststoffmaterial und eingebettet in der Erde, in schematischer Darstellung und Draufsicht zeigt; sowie

Fig. 6 den Schnitt entlang der Linie Y–Y in Fig. 5 zeigt.

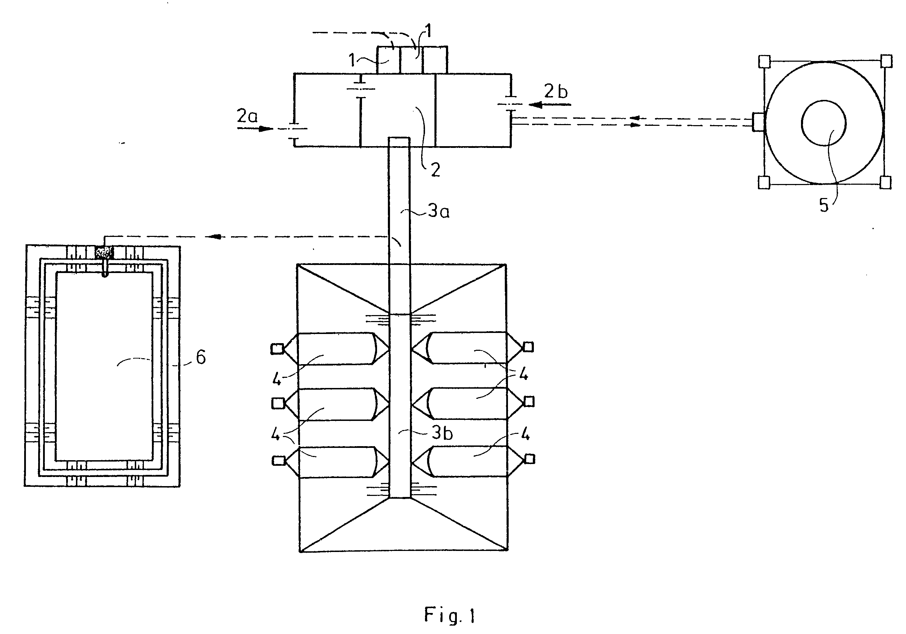

Wie aus Fig. 1 ersichtlich, sind die an sich bekannten Konstruktionsteile der Einrichtung gemäss der Erfindung folgende: die Schächte 1 dienen zum Rühren des Rohstoffes, zur Dosierung und Vorwärmung desselben und zur Rezirkulierung der im Vorgang teilnehmenden Materialien. Die Schächte 1 sind an das Hauptgebäude angeschlossen, in welchem die Pumpen, der Gaskessel und die Messinstrumente angeordnet sind. Der Pfeil 2a zeigt die Einführungsrichtung in den Behandlungs-Aufenthaltsraum bzw. in den Pumpenraum, wobei der Pfeil 2b die Einführungsrichtung in den Gasbehandlungsraum bzw. in den Gaskesselraum ausweist.

Das Hauptgebäude 2 ist über Kanäle 3a der öffentlichen Werke an dem Kanal 3b der öffentlichen Werke angeschlossen, in welchem Schlamm-, Gas- und Heizleitungen, deren Befestigungseinheiten und Messinstrumente zur Bedienung der Gärbehälter 4 angeordnet sind.

Die Gärbehälter 4 sind aus gasdichtem Kunststoffmaterial in der Weise ausgebildet, dass der zu der Fermentation (Ausfaulung) erforderliche Gärungsraum – wie er in Fig. 1 ersichtlich ist – aus dem gasdichten Kunststoffmaterial in einer realisierbaren Grösse auf sechs Gärbehälter aufgeteilt wurde, die zueinander parallelgeschaltet zusammenwirken. Das in dem Gärbehälter 4 entstehende Biogas wird einem gleichfalls aus einem gasdichten Kunststoffmaterial hergestellten trockenen Gasbehälter 5 zugeleitet, wonach dieser Trockengasbehälter 5 den erforderlichen Gasdruck für die Teilnehmer liefert. Der aus den Gärbehälter 4 herausgeführte Flüssigdünger wird als Fertigprodukt in einem ähnlich aus gasdichtem Kunststoffmaterial hergestellten geschlossenen Flüssigbiodüngerbehälter 6 hineingeführt. Dieser Flüssigbiodüngerbehälter 6 ist deswegen geschlossen, damit sich die Güte bzw. der Nährstoffgehalt des Biodüngers bis zu dessen Anwendung nicht verschlechtern.

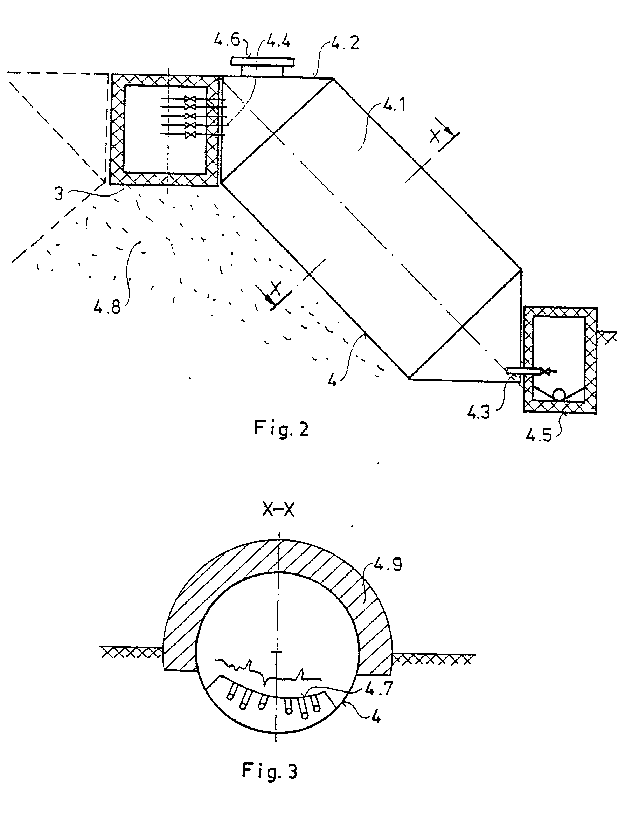

Im Sinne der Erfindung gewährt die Gruppe der Gärbehälter 4 (Ausfauler) – wie es aus Fig. 2 ersichtlich ist – das erforderliche Gärungs-Ausfaulervolumen in der Einrichtung. Wie schon erwähnt, werden die Gärbehälter 4 in solchen Abmessungen gebaut, dass sich ein günstig herstellbares Folien-Gefäss-Volumen (50–100–200 m³ usw.) ergibt. Jeder Gärbehälter 4 besteht aus einem zylindrischen Teil 4.1 und zwei daran angeschlossenen oberen bzw. unteren kegelförmigen Teilen 4.2. In dem oberen kegelförmigen Teil 4.2 ist ein gasdicht verschliessbares Mannloch 4.6 für die Montage ausgebildet. Am unteren kegelförmigen Teil 4.2 ist ein Ausleitungsrohr 4.3 angeordnet, das an einen Kanal 4.5 angeschlossen ist, der aus Stahlbeton hergestellt ist und zur Abteilung des Schlammes dient. Durch dieses Ausleitungsrohr 4.3 ist es möglich, Sand, der sich am Boden des Behälters angesammelt hat, aus dem Gärbehälter 4 zu entfernen, und zwar in der Weise, dass das Schubventil, montiert an dem Ausleitungsrohr 4.3, geöffnet wird, wodurch der Sand durch den Kanal 4.5 in den geschlossenen Flüssigbiodüngerbehälter 6 gelangt.

Die von dem Hauptgebäude 2 ausgehenden, den Schlamm einführenden, den Schlamm ausleitenden, den Schlamm rezirkulierenden, hin- und rückleitenden Rohre sowie die Zu- und Ableitungsrohre für die zur Gärung erforderliche Warmwasserheizung und das Gasabführrohr verlaufen durch die mit der Wand des Kanals 3b der öffentlichen Werke in Berührung stehende Wand 4.4 des oberen kegelförmigen Teiles 4.2. Die erwähnten Rohre sind gemeinsam in den Kanal 3b der öffentlichen Werke eingeführt, wo sie mit ihren entsprechenden Anschlüssen verbunden sind. Hier befinden sich auch diese Rohre absperrende Schiebereinrichtungen und auch die zu diesen Leitungen gehörenden Messgeräte. Die Rohrleitungen, der Heizkörper und die Fühlorgane der Heizgeräte können aus miteinander verbindbaren Teilen durch die Gärbehälter 4 (Ausfauler) in Form von elementaren Stücken eingebracht und drinnen zusammengebaut werden und gegen die der jeweiligen Technologie entsprechenden inneren Schlammströmungen bzw. durch diese verursachte Bewegungen mittels solcher Stützen 4.7 abgestützt werden, die solche Strömungen bzw. Bewegungen nicht stören (siehe Fig. 3). In der unmittelbaren Umgebung der Stützen 4.7 sind an sich bekannte Heizorgane, z.B. Rohrschlangen angeordnet.

Die Gärbehälter (Ausfauler) 4 liegen mit ihrem halben Körper in schrägen Erdaufwürfen und sind oben an dem Kanal 3b der öffentlichen Werke und unten an dem Kanal 4.5 verankert. Der obere Teil, d.h. die obere Hälfte des Gärbehälters (Ausfaulers) 4 ist mit einer separaten Wärmeisolierungsschicht 4.9 überzogen, um den Wärmeverlust auf einem geringeren Mass halten zu können. Jeder Gärbehälter (Ausfauler 4) kann nach dem Lösen der im Kanal 3b angeordneten geflanschten Montageeinheiten und nach Lösen seiner Verankerungen an der Wand des wasserableitenden Kanals 4.5 schnell und leicht ausgetauscht werden. Vor solch einem Austausch muss die innere Füllung durch die Gravitationskräfte und mittels einer Pumpe in den geschlossenen Flüssigbiodüngerbehälter übergeleitet werden. Der Austausch kann durchgeführt werden, nachdem das Ausleitungsrohr 4.3 im Gärbehälter demontiert und mit der Montagearmatur durch das obere Mannloch 4.6 herausgehoben worden ist.

Die typisierten Elemente der inneren Rohrleitungen können in gleichgrosse andere Gärbehälter eingebaut werden.

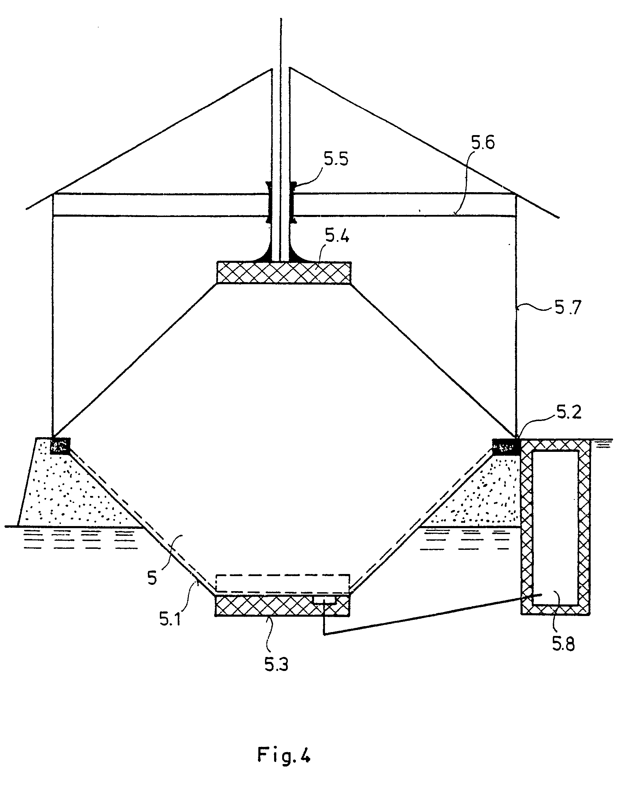

Der Trockengasbehälter 5 ist im Sinne der Erfindung (siehe Fig. 4) aus den folgenden Bestandteilen aufgebaut:

Eine gasdichte Kunststofflinse 5.1 mit entsprechenden inneren Abmessungen ist von einem Stahlbeton-Ringträger 5.2 umgeben. Die Kunststofflinse 5.1 wird von unten von einer den Anschluss für die Ein- und Ausleitungsgasrohre ermöglichenden Stahlbeton-Grundplatte 5.3 festgehalten. Es ist eine Lastplatte 5.4 vorgesehen, die für den erforderlichen Druck im Gasnetz sorgt. Die funkenfreie Bewegung dieser Lastplatte 5.4 nach oben und nach unten wird über die Führungsstange 5.5 und die diese letztere umfassende Rahmenkonstruktion 5.6 mit Haltebalken erreicht. Der gesamte Trockengasbehälter 5 ist von einer gasfesten Umhüllung 5.7 umgeben. Ein Kanal 5.8 ist vorgesehen, in welchem die Anschlüsse der ein- und ausleitenden Gasrohre, deren Armaturen, Messinstrumente, ein Kondensgefäss und ein die Rückzündung verhinderndes Gefäss angeordnet sind.

Die gasdichte Kunststofflinse 5.1, hergestellt aus Kunststoffmaterial, kann im Falle einer wesentlichen Beschädigung nach dem Lösen ihrer Verankerung an dem Stahlbeton-Ringträger 5.2, der Stahlbeton-Grundplatte 5.3 und der Stahlbeton-Lastplatte 5.4 ausgetauscht werden. Von dem Luftraum zwischen der luftfesten Umhüllung 5.7 und dem Stahlbeton-Ringträger 5.2 wird die Frostfestigkeit des feuchten Biogasraumes sichergestellt. Dieser Raum ist von der dem Kanal 5.8 zugewandten Seite der luftfesten Umhüllung 5.7 durch eine gut verschliessbare Tür zugänglich.

Der geschlossene Flüssigbiodüngerbehälter 6 besteht aus einem rechteckigen Erdaufwurf, einer unteren Kunststoff-Folienmembran 6.2 und einer oberen Folienmembran 6.3, die auf diesen rechteckigen Erdaufwurf 6.1 gelegt sind. Diese Folienmembranteile 6.2 und 6.3 verfügen über ein- und ausleitende Schlammrohr-Verschlussmontage-Einheiten und einen Schlammpegelanzeiger, die in dem Kanal 6.4 angeordnet sind. Vor diesem Kanal 6.4 ist in der gesamten Seitenlänge der oberen Folienmembrane 6.3 eine von einer Doppelfolie gebildete Öffnung 6.5 vorgesehen. Die obere Folienmembrane 6.3 liegt auf einem mittleren Längsbalken 6.6 und ist in dem Ringträger 6.6, der in dem rechteckigen Erdaufwurf liegt, gasdicht verankert.

Durch den Kanal 6.4 führt ein in der Zeichnung nicht dargestelltes Gasausleitungsrohr in den Innenraum zwischen der oberen Folienmembran 6.3 und der unteren Folienmembran 6.2 und endet an dem Längsbalken 6.6. Die Aufgabe dieses Rohres ist es, die noch in dem nachzugärenden Schlamm vorhandenen Biogase herauszuführen, um diese Gase entweder in den Trockengasbehälter 5 oder in Verbraucherleitungen einzuleiten. Der geschlossene Biodüngerbehälter 6 kann für eine Speicherung des Schlammes von 1–3 Monaten bemessen werden.

**Patentansprüche**

1. Einrichtung zur kontinuierlichen und nassen Fermentation landwirtschaftlicher Nachprodukte und Abwasserschlämme zwecks Herstellung von Biogas bzw. nährstoffhaltigem flüssigen organischen Dünger, mit wenigstens einem Gärbehälter, dessen Gasaustrittsleitungen mit wenigstens einem Trockengasbehälter in Verbindung stehen, wobei das ausfermentierte Material aus dem Gärbehälter in einen geschlossenen Biodüngerbehälter geleitet wird, dadurch gekennzeichnet, dass diejenigen Konstruktionselemente des Gärbehälters (4), des Trockengasbehälters (5) und des geschlossenen Flüssigbiodüngerbehälters (6), die mit dem zu fermentierenden Rohmaterial, mit dem Biogas bzw. dem Fertigprodukt-Biodünger in Berührung stehen, aus gasfestem Kunststoffmaterial ausgebildet sind, wobei die Behälter (4, 5, 6) mit ihrer unteren Hälfte in den Erdboden eingebettet sind, der schräg angeordnete Gärbehälter (4) mit seinem oberen und unteren Ende an oberen Zuführkanalwänden bzw. unteren Abführkanalwänden festgelegt ist und die Kunststoffwände des Trockengasbehälters (5) und des Biodüngerbehälters (6) an Ringträgern (5.2, 6.7) verankert sind.

2. Einrichtung nach Patentanspruch 1, dadurch gekennzeichnet, dass ein Ende des Gärbehälters (4) an dem Kanal (3b) der öffentlichen Werke durch Einhängung, und weiter durch verschiedene Schlamm-, Gas- und Rauchrohre, sein anderes Ende jedoch an dem den Schlamm ableitenden Kanal (4.5) mittels eines Ausleitungsrohres (4.3) verankert sind.

3. Einrichtung nach Patentanspruch 1 oder 2, dadurch gekennzeichnet, dass in dem Gärbehälter (4) ein durch ein gasdichtes Mannloch (4.6) einmontierbare Rohrleitungen fixierender Stützarm (4.7) angeordnet ist, und dass in der unmittelbaren Nähe der letztgenannten Organe Heizkörper, z.B. Rohrschlangen angeordnet sind.

4. Einrichtung nach einem der Patentansprüche 1 bis 3, dadurch gekennzeichnet, dass der Trockengasbehälter (5) über eine obere und untere Kunststoffmembran (5.1) verfügt, deren Rand an dem Stahlbeton-Ringträger (5.2) fixiert ist; dass seine Bodenwand an einer Stahlbetongrundplatte (5.3) und sein oberer Teil an einer als Druckplatte dienenden Stahlbetonlastplatte (5.4) fixiert sind, und dass diese Lastplatte (5.4) mit einer eine funkenfreie Bewegung sicherstellenden Führungsstangen (5.5) und einer diese umfassenden Rahmenkonstruktion (5.6) verbunden ist.

5. Einrichtung nach einem der Patentansprüche 1 bis 4, dadurch gekennzeichnet, dass der bedeckte Flüssigbiodüngerbehälter (6) aus einer unteren Membran (6.2) und einer oberen Membran (6.3) besteht, dass dieser Biodüngerbehälter (6) an dem Ringträger (6.7), der in einem rechteckigen Erdaufwurf (6.1) liegt, verankert ist; und dass dieser Biodüngerbehälter (6) eine zur Entfernung des schon fermentierten Düngers dienende Öffnung (6.5) aufweist, die an einer Seite aus einer Doppelfolie ausgebildet ist.

**Revendications**

1. Installation pour la fermentation continue par voie humide de sous-produits agricoles et de boues d'eaux usées envue de la production de biogaz ou d'engrais organique nourricier liquide à l'aide d'au moins un réservoir de fermentation, dont le conduits d'évacuation de gaz sont reliés avec au moins un réservoir de gaz sec, le produit fermenté étant conduit du réservoir de fermentation à un réservoir fermé d'engrais biologique, caractérisée en ce que les éléments de construction du réservoir de fermentation (4), du réservoir de gaz sec (5) et du réservoir fermé d'engrais biologique liquide (6) qui sont en contact avec le produit brut devant fermenter, avec le biogaz ou le produit fini d'engrais biologique, sont réalisés en une matière synthétique étanche aux gaz, les réservoirs (4, 5, 6) étant enfoncés dans le sol par leur moitié inféieure, le réservoir de fermentation (4) disposé en biais étant fixé par ses extrémités haute et basse aux parois supérieures du canal d'amenée ou respectivement aux parois inférieures du canal d'évacuation, et les parois en matière synthétique du réservoir de gaz sec (5) et du réservoir d'engrais biologique (6) étant ancrées à des supports annulaires (5.2, 6.7).

2. Installation selon la revendication 1, caractérisée en ce qu'une extrémité du réservoir de fermentation (4) est ancrée au canal (3b) du réseau public par suspension et, en outre, par différents tuyaux de boue, de gaz et de fumée, alors que son autre extrémité est ancrée au canal (4, 5) d'évacuation de la boue au moyen d'un tube de sortie (4.3).

3. Installation selon la revendication 1 ou 2, caractérisée en ce que, dans le réservoir de fermentation (4) est disposé un bras d'appui (4.7) fixant les tuyauteries pouvant être montées par un trou d'homme étanche aux gaz (4.6) et en ce que, des corps chauffants, tels que des serpentins, sont disposés au voisinage immédiat de ces tuyauteries.

4. Installation selon l'une des revendications 1 à 3, caractérisée en ce que le réservoir de gaz sec (5) est disposé sur une membrane haute et basse (5.1) en matière plastique et dont le bord est fixé au support annulaire en de béton armé (5.2), en ce que sa paroi de fond est fixée à une plaque de base en béton armé (5.3) et sa partie supérieure est attachée à une plaque de charge (5.4) en béton armé servant de plaque de compression et que cette plaque de charge (5.4) est reliée à une tige de guidage (5.5) assurant un déplacement exempt d'étincelles et à une structure de cadre (5.6) entourant cette tige.

5. Installation selon l'une quelconque des revendications 1 à 4, caractérisée en ce que le réservoir couvert (6) d'engrais biologique liquide comprend une membrane inférieure (6.2) et une membrane supérieure (6.3), en ce que ce réservoir (6) est ancré au support annulaire (6.7) situé dans un remblai rectangulaire (6.1) et en ce que ce réservoir d'engrais biologique (6) présente une ouverture (6.5) servant à évacuer l'engrais déjà fermenté et qui est formée d'un côté par une feuille double.

## Claims

1. Plant for the continuous and wet fermentation of agricultural by-products and waste water slurries to produce manure gas respectively multicomponent liquid organic fertilizers, comprising at least one fermentation vessel, the gas discharge conduits of which are in contact with at least one dry gas vessel, the fermented material being directed from the fermentation vessel into a closed container of biofertilizer, characterized in that the construction elements of the fermentation vessel (4), of the dry gas vessel (5) and of the closed container of liquid biofertilizer (6) which are in contact with the manure gas respectively with the end product – biofertilizer, are made of gas-tight plastics material, the vessels (4, 5, 6) being embedded with their lower half into the earthground, the inclined arranged fertmentation vessel (4) being fixed with its upper and lower end to upper entrance duct walls respectively to lower exit duct walls and the plastics wall of the dry gas vessel (5) and of the container of biofertilizer (6) are anchored at ring supports (5.2, 6.7).

2. Plant according to claim 1, characterized in that one end of the fermentation vessel (4) is anchored at the duct (3b) of the public drains through hanging and further by means of different slurry, gas and smoke pipes at the duct (4.5) discharging the slurry.

3. Plant according to claim 1 or 2, characterized in that in the fermentation vessel (4) a supporting arm (4.7) is arranged which fixes the pipelines that can be mounted through a gas-tight manhole and that next to the last mentioned devices heaters, e.g. worm pipes are arranged.

4. Plant according to one of the claims 1 to 3, characterized in that the dry gas vessel (5) has an upper and a lower plastics material membrane (5.1) the edge of which is fixed at the reinforced concrete ring support (5.2); that its floor is fixed to a reinforced concrete plate (5.3) and its upper part is fixed to a reinforced concrete plate (5.3) serving as a pressing plate, and that this load plate (5.4) is connected with a guide rod (5.5) assuring spark-free movement and with a framework construction (5.6) surrounding this guide rod (5.5).

5. Plant according to one of the claims 1 to 4, characterized in that the covered container of liquid biofertilizer (6) consists of a lower membrane (6.2) and an upper membrane (6.3), that this container of biofertilizer (6) is anchored at the ring support (6.7) which lies on a rectangular mound; and that this container of biofertilizer (6) has a gap (6.5) for removing the fermented fertilizers, and this gap (6.5) is formed from double foil on one side.

Fig.1

Fig. 2

X–X

Fig. 3

3/4

Fig. 4

4/4

Fig. 5

Y — Y

Fig. 6